# EUROPEAN PATENT SPECIFICATION

(11) **EP 3 001 985 B1**
(45) Date of publication and mention of the grant of the patent: **27.10.2021**
(21) Application number: 15194468.3
(22) Date of filing: 27.09.2007
(51) Int. Cl.: A61F 2/915

(54) **STENT AND STENT DELIVERY DEVICE**
STENT UND STENTVERABREICHUNGSVORRICHTUNG
ENDOPROTHÈSE VASCULAIRE ET SON DISPOSITIF DE MISE EN PLACE

(30) Priority: 29.09.2006 JP 2006269201
(43) Date of publication of application: 06.04.2016
(62) Divisional of application: 07829145.7
(73) Proprietor: Terumo Kabushiki Kaisha, Tokyo 151-0072 (JP)
(72) Inventor: GOTO, Hiroki, Kanagawa, 259-0151 (JP)
(74) Representative: Casalonga

(56) References cited:
- EP-A1- 1 428 549
- WO-A2-02/060344
- WO-A2-2004/058105
- FR-A1- 2 781 143
- US-A1- 2003 014 102

## Description

### Technical Field

The present invention relates to a stent that is implanted in lumens of a living body such as the blood vessel, the bile duct, the trachea, the esophagus, the ureter, and the like to improve a stenosed portion or a closed portion generated in the lumens. The present invention also relates to a stent delivery device.

### Background Art

To cure various diseases that are caused when the blood vessel or lumens are stenosed or closed, the stent is a tubular medical appliance to be implanted in lumens of the living body to expand a stenosed portion or a closed portion to secure the lumen thereof.

The stent is inserted into the body from the outside. Therefore the stent is so constructed that its diameter is small when it is inserted into the body and is enlarged at a desired stenosed or closed portion by expanding it and that it keeps an expanded state of the lumen thereof.

The stent is cylindrical and made of a metal wire or a processed metal pipe. After the stent is mounted on a catheter or the like by decreasing its diameter, it is inserted into the body. Thereafter the stent is expanded at a desired portion by using an expanding method and fixed to an inner wall of the lumen of the desired portion, with the stent in close contact therewith to maintain the configuration of the lumen. The stent is classified into a self-expandable stent and a balloon expandable stent in dependence on the function thereof and an implantation method. The balloon expandable stent which itself has no expanding function is secured at a desired portion. Then, a balloon provided in the stent is inflated to expand (plastically deform) the stent by an expansive force of the balloon so that the stent is fixed to the inner surface of the desired lumen with the stent in close contact therewith. It is necessary to perform an operation of expanding the stent of this type in implanting it to the desired portion of the living body.

The balloon expandable stent is mostly used as the stent to be used to cure blood vessels and particularly the coronary arteries. The stent is demanded to have an axially flexible construction to cope with a lot of cases.

The balloon expandable stent is classified into a closed cell type and an opened cell type in dependence on the configuration of a stratum thereof. The balloon expandable stent of the opened cell type has an advantage that it is flexible. Thus the balloon expandable stent of the opened cell type is favorably implanted in a desired portion because it is capable of flexibly following a travel direction of a blood vessel and its configuration. Thereby it is possible to prevent the blood vessel from being stimulated. But the balloon expandable stent of the opened cell type has a disadvantage that the stratum thereof flares outward. On the other hand, the balloon expandable stent of the closed cell type has an advantage that the stratum thereof does not flare outward, but is incapable of flexibly following the travel direction of the blood vessel and its configuration. The balloon expandable stent of the opened cell type and the closed cell type have both the advantage and the disadvantage. Thus it is necessary to use the balloon expandable stent of the opened cell type or the closed cell type in dependence on the travel direction and configuration of the blood vessel.

The balloon expandable stent of the opened cell type is proposed by the present applicant and disclosed in Japanese Patent Application Laid-Open No.2002-136601.

The closed cell-type stent is disclosed in WO96/03092 and Japanese Patent Application Laid-Open No.10-503676 corresponding to WO96/03092.

But in this closed cell-type stent, the connection portions are disposed all over. Thus this stent lacks flexibility and has a design not suitable for following the inside of a blood vessel and for being disposed at a curved portion.

Even in the balloon expandable stent of the opened cell type disclosed in Japanese Patent Application Laid-Open No.2002-136601, the opened cell portion has a sufficient expanded-state retention force. But it is desirable that the stent has a higher expanded-state retention force. It is also desirable that the stent has a low degree of a length change in its axial direction when it expands and has a higher follow-up performance for organs. EP 1 428 549 A1 discloses a stent with a tubular member comprising cylindrical lumen size-holding portions for holding a lumen of a living body open, and connecting portions for longitudinally connecting the lumen size-holding portions adjacent to one another. The lumen size-holding portions are configured into repeated patterns running vertically in the developed conditions thereof. Each of the repeated patterns comprises a first curved pattern portion having axis extending in a lateral direction and second and third curved pattern portions having axes extending in vertical directions. The upper and lower first curved pattern portions adjacent to one another are connected at one end thereof by the second curved pattern portion, and at the other end by the third curved pattern portion. The stent is excellent in flexibility and trackability to lumens. Further, the stent is capable of being passed through three-dimensionally meandering lumens and possesses a sufficient strength for holding a widened size of a lumen

It is an object of the present invention to provide a stent, to be implanted in a living body, which has a low degree of a length change in its axial direction when it expands, a high follow-up performance for organs and a high expanded-state retention force; and a stent delivery device.

### Disclosure of Invention

The above-described object is achieved by a stent as set forth in the appended claims, any other embodiment is cited as an example.

A stent is formed substantially as a tube having a form in which a plurality of wavy annular members is arranged adjacently to each other in an axial direction of the stent with the adjacent wavy annular members connected with each other. The stent has a diameter whose dimension is so set that the stent can be inserted into a lumen inside a living body. The stent can be expanded when a force spreading radially from an inside of the tube is applied thereto. The wavy annular member has parallel straight-line portions extended in parallel with the axis of the stent before and after the stent expands. The stent has connection portions each connecting opposed ends of the parallel straight-line portions of the adjacent wavy annular members to each other.

Also, the above-described object is achieved by a stent delivery device described below.

A stent delivery device comprises a tubular shaft body; a balloon, foldable and expandable, which is disposed at a distal end of the shaft body; and a stent which is mounted on the folded balloon, with the stent covering the balloon and is expanded owing to expansion of the balloon. As the above-described stent, the above-described stent is used.

### Brief Description of Drawing

Fig. 1 is a front view of a stent of one embodiment of the present invention to be implanted in a living body.
Fig. 2 is a development view of the stent shown in Fig. 1.
Fig. 3 is a partly enlarged view of Fig. 2.
Fig. 4 is a development view of the stent shown in Fig. 1 at the time when the stent is manufactured.
Fig. 5 is a partly enlarged view of Fig. 4.
Fig. 6 is a development view of a stent of another embodiment of the present invention to be implanted in a living body at the time when the stent is manufactured.
Fig. 7 is a partly enlarged view of Fig. 6.
Fig. 8 is a development view of a stent of another embodiment of the present invention to be implanted in a living body at the time when the stent is manufactured.
Fig. 9 is a development view of a stent of another embodiment of the present invention to be implanted in a living body at the time when the stent is manufactured.
Fig. 10 is a development view of a stent of another embodiment of the present invention to be implanted in a living body at the time when the stent is manufactured.
Fig. 11 is a front view showing a stent delivery device of an embodiment of the present invention.
Fig. 12 is a partly enlarged sectional view showing a distal portion of the stent delivery device shown in Fig. 11.
Fig. 13 is an explanatory view for explaining the operation of a stent delivery device of an embodiment of the present invention.

### Best Mode for Carrying Out the Invention

The stent of the present invention to be implanted in a living body is described below by using the following preferred embodiments.

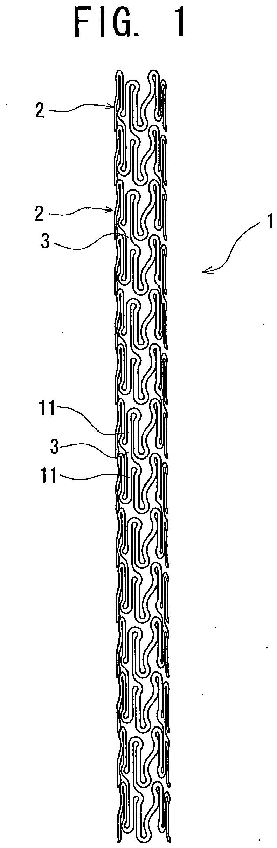

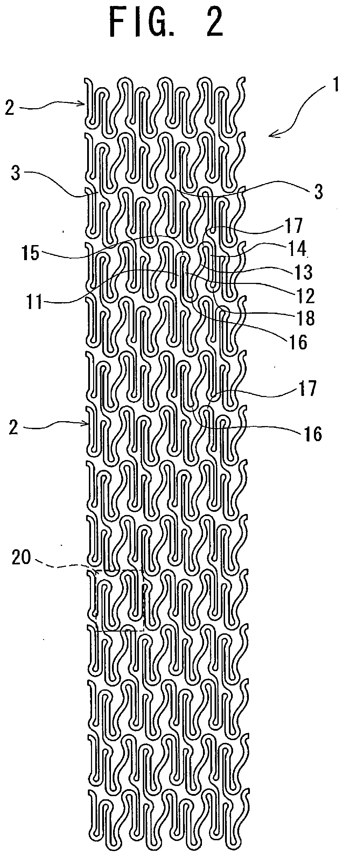

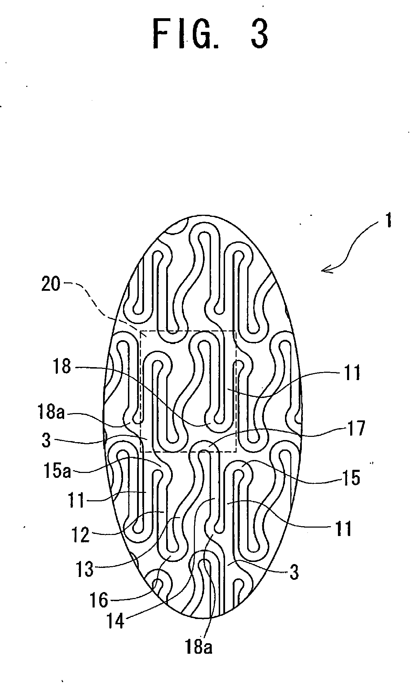

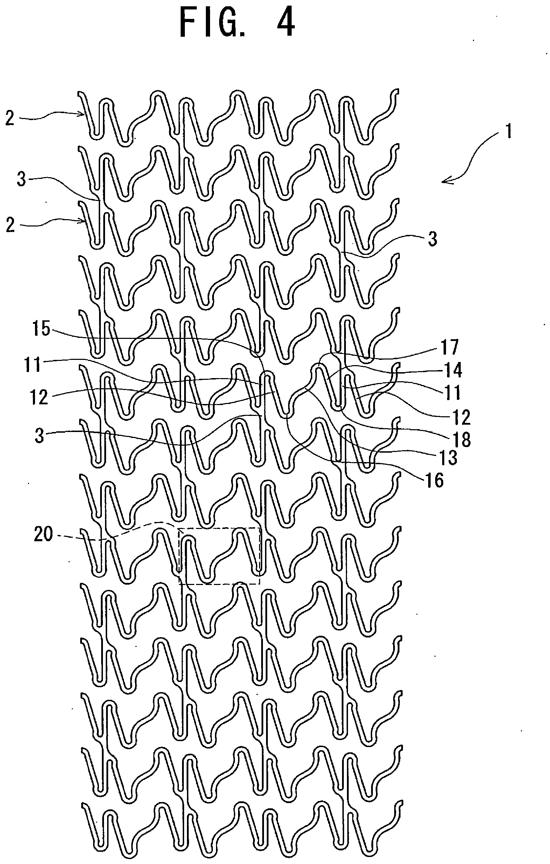

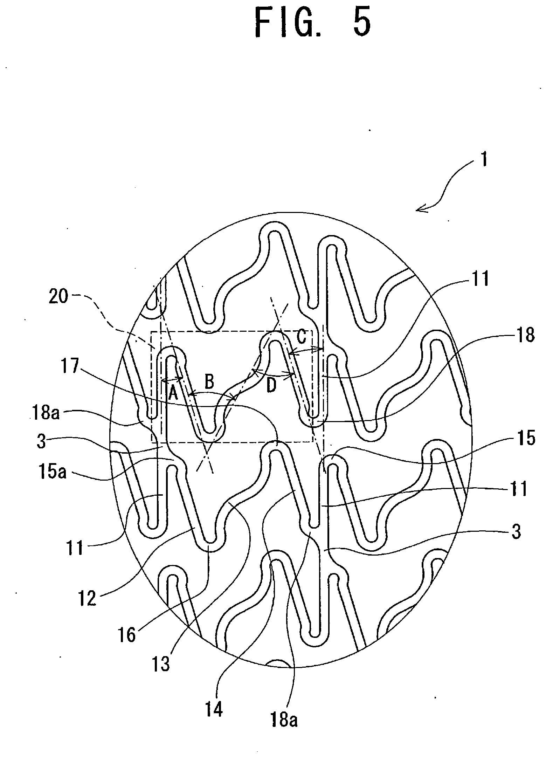

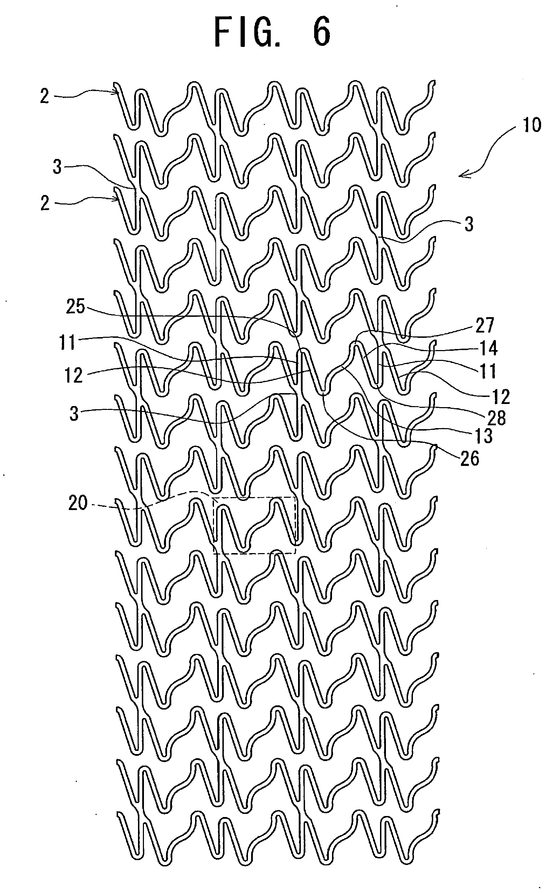

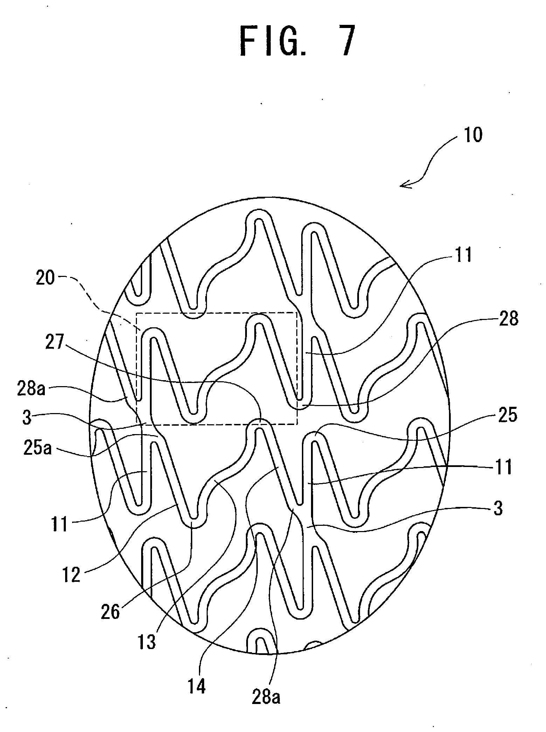

Fig. 1 is a front view of a stent of one embodiment of the present invention to be implanted in a living body. Fig. 2 is a development view of the stent shown in Fig. 1. Fig. 3 is a partly enlarged view of Fig. 2. Fig. 4 is a development view of the stent shown in Fig. 1 at the time when the stent is manufactured. Fig. 5 is a partly enlarged view of Fig. 4. Fig. 6 is a development view of a stent of another embodiment of the present invention to be implanted in a living body at the time when the stent is manufactured. Fig. 7 is a partly enlarged view of Fig. 6.

A stent 1 of the present invention to be implanted in a living body is formed substantially as a tube which has a form in which a plurality of wavy annular members 2 is arranged adjacently to each other in an axial direction of the stent 1 with the adjacent wavy annular members 2 connected with each other, has a diameter whose dimension is so set that the stent 1 can be inserted into a lumen inside a living body, and can be expanded when a force spreading radially from an inside of the tube is applied thereto. The wavy annular member 2 has parallel straight-line portions 11 extended in parallel with the axis of the stent 1 before and after the stent 1 expands. The stent 1 has connection portions 3 each connecting ends of the parallel straight-line portions 11 of the adjacent wavy annular members 2 to each other.

The stent 1 is formed substantially as a tube and has a diameter whose dimension is so set that the stent 1 can be inserted into a lumen inside a living body. The stent 1 can be expanded when a force spreading radially outward from the inside of the tube is applied thereto. The stent 1 is a so-called balloon expandable stent.

As shown in Figs. 1 and 2, the stent 1 of the present invention has a plurality of the wavy annular members 2 arranged adjacently to each other in the axial direction thereof, with the adjacent wavy annular members connected with each other.

The number of the wavy annular members 2 forming the stent 1 shown in Figs. 1 through 5 is set to 14. The number of the wavy annular members 2 is different in dependence on the length of the stent and is set favorably to the range of 4 to 50 and more favorably to the range of 10 to 35.

Each of the wavy annular members 2 has a plurality of one-end side bent portions 15, 17 each having an apex at one-end side of the stent 1 in an axial direction thereof and a plurality of other-end side bent portions 16, 18 each having an apex at the other-end side of the stent 1 in the axial direction thereof. Each of the wavy annular members 2 is composed of an annularly continuous endless wavy line element. The one-end side bent portions 15, 17 and the other-end side bent portions 16, 18 are formed alternately. The number of the one-end side bent portions 15, 17 and that of the other-end side bent portions 16, 18 are set equally to each other.

The total of the one-end side bent portions 15, 17 of one wavy annular members 2 shown in Figs. 1 through 4 is set to eight. Similarly, the total of the other-end side bent portions 16, 18 of one wavy annular members 2 is also set to eight. The number of the one-end side bent portions of one wavy annular members 2 and that of the other-end side bent portions thereof are set favorably to the range of 4 to 12 and especially favorably to the range of six to ten. The length of the wavy annular member 2 in the axial direction of the stent is set favorably to the range of 0.5 to 2.0mm and especially favorably to the range of 0.9 to 1.5mm.

As shown in Figs. 1 through 4, the wavy annular member 2 includes a plurality of modified M-shaped linear portions 20, continuous with each other, each of which has four linear portions composed of a parallel straight-line portion 11; a first inclined straight-line portion 12 which is connected with one end of the parallel straight-line portion 11 through the bent portion 15 (15a) and becomes oblique to the axis of the stent 1 at a predetermined angle at least when the stent 1 expands; an inclined linear portion (in this embodiment, inclined curved line portion) 13 which is connected with one end of the first inclined straight-line portion 12 through the bent portion 16 and extended obliquely at a predetermined angle to the axis of the stent; and a second inclined straight-line portion 14 connected with one end of the inclined curved line portion 13 through the bent portion 17 and becomes oblique to the axis of the stent 1 at a predetermined angle at least when the stent 1 expands. The adjacent modified M-shaped linear portions 20 are connected with the bent portion 18 (18a) which connects one end of the second inclined straight-line portion 14 and the other end of the parallel straight-line portion 11 with each other, thus constructing the endless wavy annular member 2. Therefore it is possible to restrain the wavy annular member 2 from shortening in the axial length thereof when the stent expands and impart a sufficient expanded-state retention force to the wavy annular member 2.

In the stent 1 of this embodiment, as shown in Fig. 3 when the stent is compressed) and Fig. 5 (at a stent-manufacturing time), the wavy annular member 2 includes a plurality of modified M-shaped linear portions 20, continuous with each other, each of which has four linear portions composed of the parallel straight-line portion 11; the first inclined straight-line portion 12 which is connected with one end of the parallel straight-line portion 11 through the bent portion 15 (15a), is almost parallel (before the stent expands) with the axis of the stent, and becomes oblique to the axis of the stent 1 at a predetermined angle when the stent 1 expands; the inclined curved line portion 13 which is connected with one end of the first inclined straight-line portion 12 through the bent portion 16 and extended obliquely at a predetermined angle to the axis of the stent; and the second inclined straight-line portion 14 which is connected with one end of the inclined curved line portion 13 through the bent portion 17, is almost parallel (before the stent expands) with the axis of the stent, and becomes oblique to the axis of the stent 1 at a predetermined angle when the stent 1 expands. That is, the first inclined straight-line portion 12 and the second inclined straight-line portion 14 are almost parallel with the axis of the stent 1 before the stent expands, in other words, when the stent is compressed. Therefore when the stent is compressed, it is possible to make the outer diameter thereof small.

More specifically, as shown in Figs. 3 and 5, the first inclined straight-line portion 12 and the second inclined straight-line portion 14 are almost parallel with the axis of the stent before the stent expands. When the stent expands, the first inclined straight-line portion 12 and the second inclined straight-line portion 14 extend in the same direction obliquely to the axis of the stent 1. In the stent 1 of this embodiment, when the stent expands, the first inclined straight-line portion 12 and the second inclined straight-line portion 14 extend obliquely to the axis of the stent and are almost parallel with each other. The inclined curved line portion 13 extends obliquely to the axis of the stent even before the stent expands. When the stent expands, the inclined curved line portion 13 extends obliquely to the axis of the stent in a direction different from the direction in which the first inclined straight-line portion 12 and the second inclined straight-line portion 14 extend.

The number of the one-end side bent portions and the other-end side bent portions of the modified M-shaped linear portions 20 is two respectively, as shown in Figs. 1 through 4. A separated distance (in other words, the width of the modified M-shaped linear portion 20 in the inclined curved line portion) between the other-end side bent portion 16 and the one-end side bent portion 17 in the direction orthogonal to the axis of the stent 1 is set larger than a separated distance (in other words, the width of the modified M-shaped linear portion 20 in the first inclined straight-line portion) between the one-end side bent portion 15 and the other-end side bent portion 16 in the direction orthogonal to the axis of the stent 1 and a separated distance (in other words, the width of the modified M-shaped linear portion 20 in the second inclined straight-line portion) between the one-end side bent portion 17 and the other-end side bent portion 18 in the direction orthogonal to the axis of the stent 1. By disposing the inclined curved line portion 13 at a wide portion, a sufficient expanded-state retention force is imparted to the wavy annular member 2.

As shown in Fig. 3, in the wavy annular member 2, the bent portion 17 disposed at the one-end side of the inclined curved line portion 13 is projected in a larger amount than the other one-end side bent portion 15 toward the one-end side in the axial direction of the stent Similarly in the wavy annular member 2, the bent portion 16 disposed at the other-end side of the inclined curved line portion 13 is projected in a larger amount than the other other-end side bent portion 18 toward the other-end side in the axial direction of the stent.

In the stent 1 of this embodiment, one wavy annular member 2 is composed of four modified M-shaped linear portions 20. It is preferable that one wavy annular member 2 is composed of three to five modified M-shaped linear portions 20.

In the stent (Figs. 1 through 3) at the time of the compression, each of the wavy annular members 2 has a plurality of the one-end side bent portions 15, 17 each having the apex at the one-end side of the stent 1 in the axial direction thereof and a plurality of the other-end side bent portions 16, 18 each having the apex at the other-end side of the stent 1 in the axial direction thereof. The apex of at least one of the one-end side bent portions 17 of the wavy annular member 2 penetrates to some extent into a space formed between the other-end side bent portions 16 of the adjacent wavy annular member 2. Similarly the apex of at least one of the other-end side bent portion 16 of the wavy annular member 2 penetrates to some extent into a space formed between the one-end side bent portions 17 of the adjacent wavy annular member 2. Therefore the wavy annular member 2 has a sufficient expanded-state retention force at the time of the expansion.

It is preferable that the length of the parallel straight-line portion 11 is set shorter than that of the first inclined straight-line portion 12, the inclined curved line portion 13, and the second inclined straight-line portion 14. By making the length of the parallel straight-line portion short, it is possible to enhance the expanded-state retention force.

It is preferable that the bent portion 15 (15a) connecting one end of the parallel straight-line portion 11 of the wavy annular member 2 and the first inclined straight-line portion 12 thereof to each other is rounded. By so doing, it is possible to disperse a strain at the time of the expansion and secure a higher safety rate. It is preferable that the bent portion 15 (15a) connecting one end of the parallel straight-line portion 11 of the wavy annular member 2 and the first inclined straight-line portion 12 thereof to each other is rounded and that the bent portion 18 (18a) connecting the other end of the parallel straight-line portion 11 of the wavy annular member 2 and the second inclined straight-line portion 14 thereof to each other is also rounded. By so doing, it is possible to disperse a strain at the time of the expansion and secure a higher safety rate. It is preferable that the other bent portions 16, 17 are also rounded.

As described above, it is preferable that the above-described bent portions are rounded. The configuration of the bent portions is not limited to the above-described one, but it is possible that the bent portions are not rounded, as in the case of a stent 10 shown in Figs. 6 and 7. The stent 10 is different from the above-described stent 1 in the configuration of the above-described bent portions and the number of the wavy annular members 2. By not rounding the bent portions, it is possible to make the outer diameter small at the compressed time and is thus advantageous in inserting the stent into an organ (for example, blood vessel) inside a living body having a small diameter.

As shown in Fig. 5, at the time of manufacturing the stent (at the expansion time of the stent), it is preferable that the ratio (B/A) of an internal angle B formed between the first inclined straight-line portion 12 and the inclined curved line portion 13 to an internal angle A formed between the parallel straight-line portion 11 and the first inclined straight-line portion 12 is set to not less than 1.8. By setting the ratio (B/A) to the above-described range, the wavy annular member 2 has a sufficient expanded-state retention force at the time of the expansion. It is especially favorable that the ratio (B/A) of the internal angle B to the internal angle A is set to 1.8 to 2.5.

At the time of manufacturing the stent (at the expansion time of the stent), it is preferable that the ratio (D/C) of an internal angle D formed between the second inclined straight-line portion 14 and the inclined curved line portion 13 to an internal angle C formed between the parallel straight-line portion 11 and the second inclined straight-line portion 14 is set to not less than 1.8. By setting the ratio (D/C) to the above-described range, the wavy annular member 2 has a sufficient expanded-state retention force at the time of the expansion. It is especially favorable that the ratio (D/C) of the internal angle D to the internal angle C is set to 1.8 to 2.5.

In the stent 1 of this embodiment, the apexes of the one-end side bent portions 15, 17 and the apexes of the other-end side bent portions 16, 18 are almost aligned to each other along the axial direction of the stent.

The adjacent wavy annular members 2 are connected to each other with the connection portions 3. In the stent 1 of this embodiment, ends of the parallel straight-line portions 11 of the adjacent wavy annular members 2 are proximate to each other and connected to each other with the short connection portions 3. Therefore the distance between the adjacent wavy annular members 2 is short, which greatly reduces the formation of a portion having a low expansive force.

In the stent 1 of this embodiment, as shown in Figs. 1 through 4, two parallel straight-line portions 11 connected to each other with the connection portion 3 are almost aligned to each other. Therefore it is possible to prevent the stent from being shortened between the adjacent wavy annular members when the stent expands. The stent 1 has a plurality of the connection portions 3 connecting the adjacent wavy annular members to each other. Therefore the adjacent wavy annular members are not accidentally spaced from each other, which allows the entire stent to display a sufficient expansive force.

More specifically, as shown in Figs. 3 and 5, in the adjacent wavy annular members 2, the bent portion 18a connecting the other end of the parallel straight-line portion 11 and the second inclined straight-line portion 14 to each other and the bent portion 15a connecting the one end of the parallel straight-line portion 11 and the first inclined straight-line portion 12 to each other are connected to each other with the short connection portion 3. Two parallel straight-line portions 11 connected to each other with the connection portion 3 are continuously aligned to each other.

In this embodiment, there is no portion where not less than two (in other words, three or more) parallel straight-line portions 11 axially continuous with each other are connected and integrated with each other with the connection portion. That is, the connection portion 3 connects only two parallel straight-line portions 11 to each other, and there is no portion where three parallel straight-line portions 11 are integral with each other. Therefore a load generated when one wavy annular member has changed its configuration to follow the deformation of a blood vessel can be prevented from being directly (or linearly) transmitted to an unadjacent wavy annular member, which allows the wavy annular members to display expansion function thereof individually.

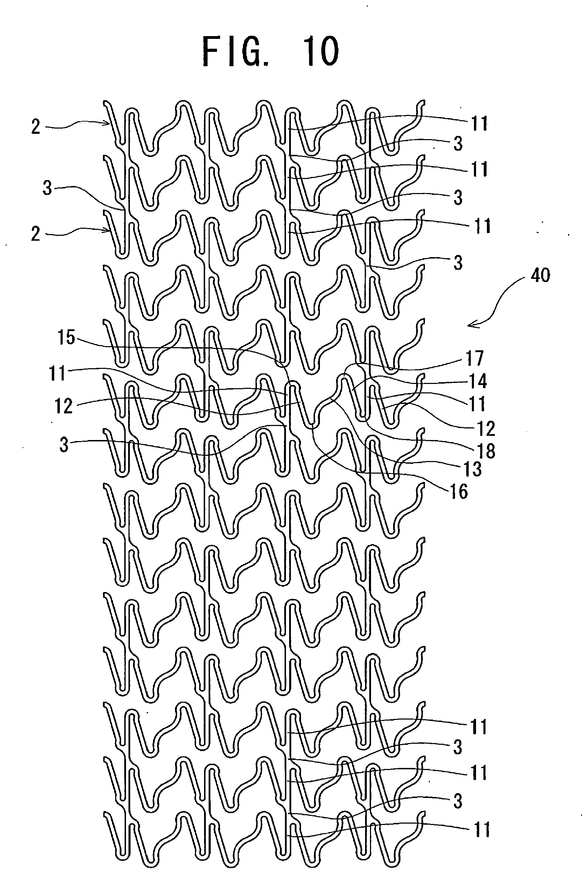

Like a stent 40 shown in Fig. 10, only both ends of the stent 40 may be provided with a portion where axially continuous three parallel straight-line portions 11 are connected and integral with each other with two connection portions 3. By so doing, it is possible to enhance the strength (expanded-state retention force) of both ends of the stent. In the stent 40, four portions where the parallel straight-line portions 11 are connected and integrated with each other by the connection portions 3 are provided at both ends thereof with the four portions forming almost an equal angle with respect to the axis of the stent. In the opposed two of the four portions, three parallel straight-line portions 11 axially continuous with each other are connected and integrated with each other by two connection portions 3.

The connection portions 3 adjacent to each other in the axial direction of the stent 1 are shifted from each other in a direction orthogonal to the axis of the stent. Therefore there is no portion where not less than two (three or more) parallel straight-line portions 11 are connected with each other with the connection portion. Therefore a load generated when one wavy annular member has changed its configuration to follow the deformation of a blood vessel can be prevented from being directly (or linearly) transmitted to an unadjacent wavy annular member, which allows the wavy annular members to display expansion function thereof individually.

The stent 1 has a plurality of connection portions 3 connecting the adjacent wavy annular members 2 to each other. Therefore the adjacent wavy annular members 2 are not spaced from each other accidentally and thus the entire stent displays a sufficient expansive force. Only one connection portion 3 may be formed between the adjacent wavy annular members 2. The length of the connection portion 3 in the axial direction of the stent 1 is set to favorably not more than 1.0mm and to especially favorably to the range of 0.1 to 0.4mm.

The stent 1 has two connection portions 3 connecting the adjacent wavy annular members 2 to each other. The two connection portions 3 are provided at opposed positions. The connection portions 3 are so disposed that they are not continuous with each other in the axial direction of the stent 1. More specifically, in the stent 1 of this embodiment, as shown in Figs. 1 through 4, two connection portions 3 are provided at positions where two connection portions 3 confront each other. Two connection portions 3 axially adjacent to each of these two connection portion 3 confront each other and are shifted from each of these two connection portion 3. These two connection portions 3 are shifted from each of axially adjacent two connection portion 3 at about 90 degrees with respect to the axis of the stent 1.

After the stent 1 is formed in a state shown by the development of Fig. 4 in which the stent 1 has a diameter larger from a state of the stent 1 shown in Figs. 1 and 2 in which the stent 1 has a smaller diameter, the stent 1 is mounted on an expandable balloon of an appliance by decreasing the diameter of the stent 1. By expanding the balloon, the diameter of the stent 1 is increased to a state in which the diameter thereof is larger than the diameter thereof in the state shown in Fig. 4.

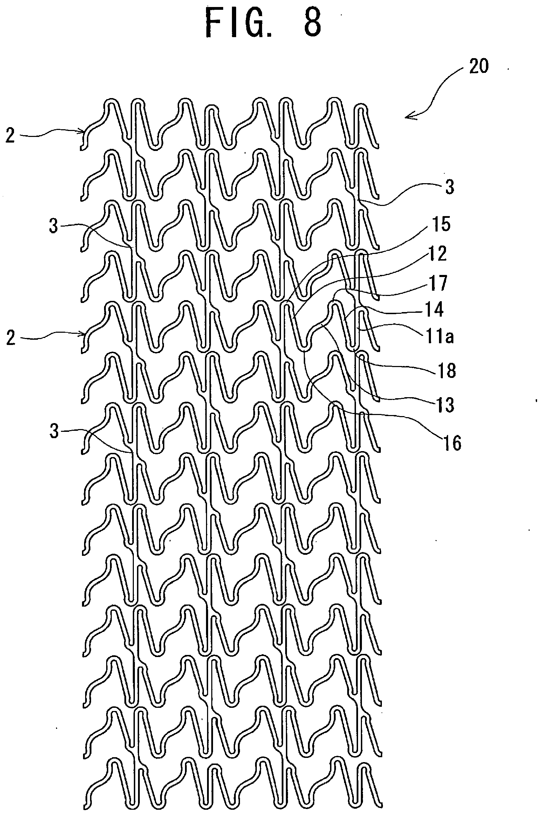

In the stents of all of the embodiments, the length of the parallel straight-line portion 11 is set shorter than that of the first inclined straight-line portion 12, the inclined curved line portion 13, and the second inclined straight-line portion 14. Although this length relationship is preferable, this length relationship does not necessarily have to be satisfied. For example, as in the case of a stent 20 shown in Fig. 8, the length of a parallel straight-line portion 11a may be almost equal to that of the first inclined straight-line portion 12, the inclined linear portion (inclined curved line portion) 13, and the second inclined straight-line portion 14. By making the length of the parallel straight-line portion 11a almost equal to that of the first inclined straight-line portion 12, the inclined linear portion (inclined curved line portion) 13, and the second inclined straight-line portion 14 as in the case of the stent 20 of this embodiment, the flexibility of the stent can be enhanced.

In the stent 20 of this embodiment, the one-end side bent portion 15 and the one-end side bent portion 17 (more specifically, the one-end side bent portion not connected by the connection portion) are disposed at almost the same position in the axial direction of the stent and penetrate into the adjacent wavy annular member 2 to some extent. Similarly the other-end side bent portion 16 and the other-end side bent portion 18 (more specifically, the other-end side bent portion not connected by the connection portion) are disposed at almost the same position in the axial direction of the stent and penetrate into the adjacent wavy annular member 2 to some extent. That is, except the bent portions connected by the connection portion, waves of the wavy annular member 2 have almost the same size. The wavy annular members 2 do not have projected bent portions.

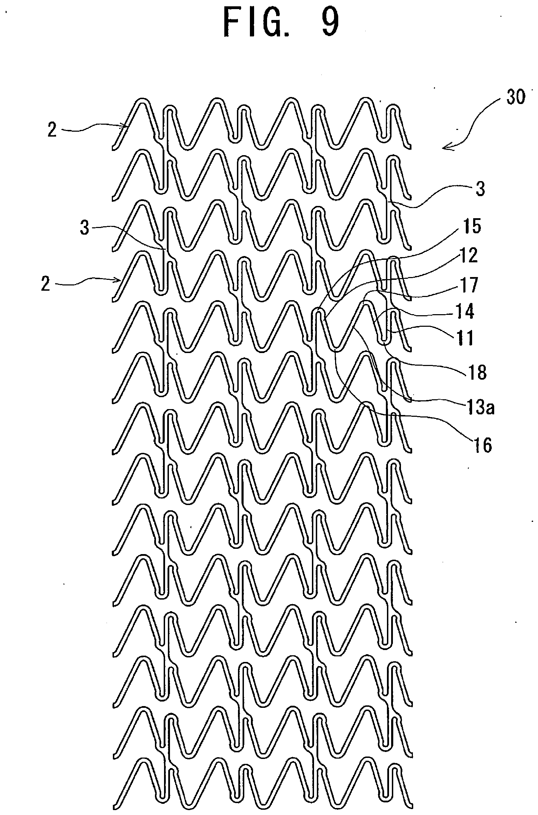

In the stents of all the above-described embodiments, the inclined linear portion 13 is formed as the inclined curved line portion. Although this mode is preferable, as in the case of a stent 30 of an embodiment shown in Fig. 9, the inclined linear portion may be a third inclined straight-line portion 13a.

The stents of all the above-described embodiments are formed substantially as a tube and has a diameter whose dimension is so set that the stent 1 can be inserted into a lumen inside a living body. The stent 1 can be expanded when a force spreading radially outward from the inside of the tube is applied thereto. The stent 1 is a so-called balloon expandable stent.

It is preferable that the material of the balloon expandable stent has a certain degree of compatibility with the living body. For example, it is possible to use stainless steel, tantalum or tantalum alloys, platinum or platinum alloys, gold or gold alloys, cobalt based alloys, a cobalt-chrome alloy, a titanium alloy, and a niobium alloy. It is preferable to plate the stent with a noble metal such as gold and platinum after the stent is fabricated into a final shape. As the stainless steel, SUS 316L most corrosion-resistant can be preferably used.

It is preferable to anneal the stent after it is fabricated into the final shape. Annealing improves the flexibility and plasticity of the entire stent. Thereby the stent can be favorably implanted at a curved portion of a blood vessel. As compared with a non-annealed stent, the annealed stent has a lower force of restoring to an original state after it is expanded, and especially has a lower force of restoring to an original linear state when it is expanded at the curved portion of the blood vessel. This minimizes physical stimulation to the inner wall of the curved portion of the blood vessel, thus reducing the cause of a recurrence of stenosis. It is preferable to anneal the stent by heating it to 900 to 1200°C in an inert gas atmosphere (e.g., a mixture gas of nitrogen and hydrogen) so that no oxide film is formed on the surface of the stent and then slowly cooling it.

The stent has a diameter favorably 0.8 to 1.8 mm and more favorably 0.9 to 1.6 mm in an unexpanded state. The stent 1 has a length favorably 8 to 40 mm in an unexpanded state. It is preferable that each wavy annular members 2 has a length of 8 to 25 mm.

The stent is shaped by removing portions other than a frame structure from a tube (more specifically, metal pipe). More specifically, the stent is formed by removing unnecessary portions from the metal pipe by an etching process, known as photo-fabrication, using masks and chemicals; electric discharge machining using a die; and cutting processing (for example, mechanical polishing, laser cutting processing). In addition, it is preferable to polish edges of the frame structure by chemical polishing or electrolytic polishing after the frame structure is formed.

The stent of the present invention may be coated with a material suitable for the living body on its inner surface, outer surface or inner and outer surfaces. As the material suitable for the living body, synthetic resin and metal suitable for the living body can be used. The following inactive metals are used to coat the surface of the stent: gold by an electroplating method, stainless steel by an evaporation method, silicon carbide, diamond-like carbon, plated titanium nitride, and plated gold by a sputtering method. As the synthetic resin, the following thermoplastic resins or thermosetting resins can be used: polyolefin (for example, polyethylene, polypropylene, ethylene-propylene copolymer), polyvinyl chloride, ethylene-vinyl acetate copolymer, polyamide elastomer, polyurethane, polyester, fluorocarbon resin, silicone resin. It is preferable to use polyolefin, polyamide elastomer, polyester, polyurethane, silicone resin. A resin decomposable in the living body (polylactic acid, polyglycolic acid, polylactic acid-polyglycolic acid copolymer) is also favorable. It is preferable that a film of the synthetic resin is soft to such an extent as not to prevent a frame constituting the stent from being curved. The thickness of the film of the synthetic resin is set favorably to the range of 3 to 300 *µ* m and more favorably in the range of 5 to 100 *µ* m.

As the method of thinly coating the surface of the stent with the synthetic resin, it is possible to use a method of inserting the stent into the melted synthetic resin or into the synthetic resin dissolved in a solution. It is also possible to use a chemical evaporation method of polymerizing a monomer over the surface of the pipe made of the super-elastic metal. In the case where the surface of the stent is coated very thinly with the synthetic resin, the use of a dilute solution or the chemical evaporation method is preferable. To improve the quality of the material suitable for the living body to a higher extent, the resinous film may be coated with an anti-thrombus material or the anti-thrombus material may be fixed to the resinous film. As the anti-thrombus material, known various resins can be used singly or as a mixture thereof. For example, polyhydroxyethyl methacrylate, a copolymer of hydroxyethyl methacrylate and styrene (for example, HEMA-St-HEMA block copolymer) can be preferably used.

An embodiment of the stent delivery device of the present invention will be described below.

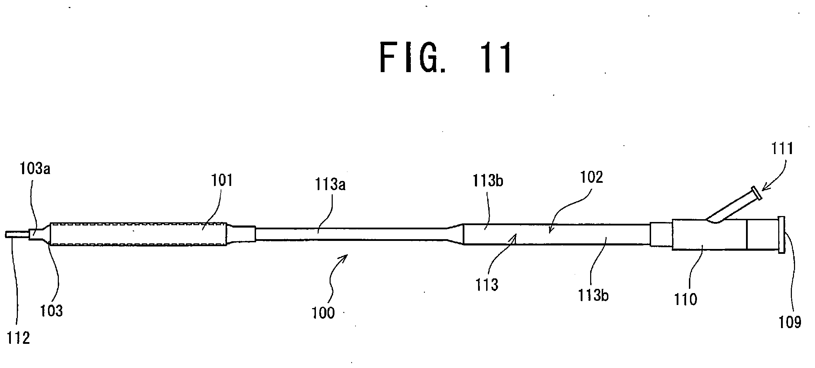

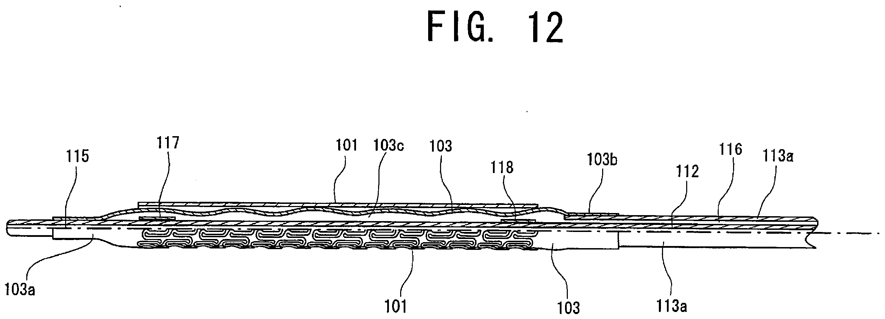

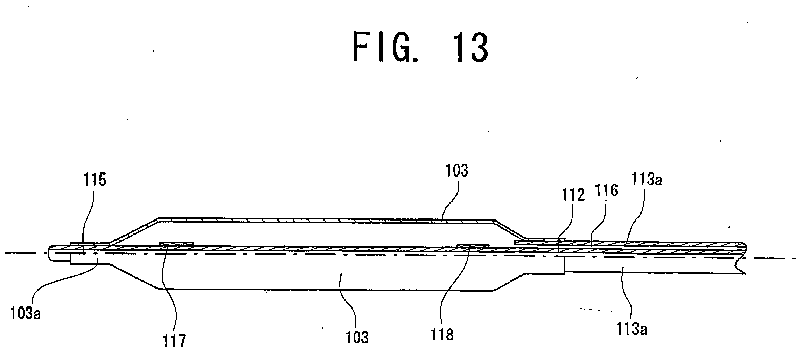

Fig. 11 is a front view showing a stent delivery device of an embodiment of the present invention. Fig. 12 is a partly enlarged sectional view showing a distal portion of the stent delivery device shown in Fig. 11. Fig. 13 is an explanatory view for explaining the operation of an stent delivery device of an embodiment of the present invention.

A stent delivery device 100 of the present invention has a tubular shaft body 102; a balloon 103, foldable and expandable, which is disposed at a distal end of the shaft body 102; and a stent 101 mounted on the folded balloon 103, with the stent 101 covering the balloon 103. The stent 101 is expanded owing to the expansion of the balloon 103.

Similarly to the above-described stent 1, the stent 101 is formed substantially as a tube which has a form in which a plurality of wavy annular members 2 is arranged adjacently to each other in an axial direction of the stent 101 with the adjacent wavy annular members 2 connected with each other, has a diameter whose dimension is so set that the stent 101 can be inserted into a lumen inside a living body, and can be expanded when a force spreading radially from an inside of the tube is applied thereto. The wavy annular member 2 has parallel straight-line portions 11 extended in parallel with the axis of the stent 101 before and after the stent 101 expands. As the stent 101, a stent having connection portions 3 each connecting ends of the parallel straight-line portions 11 of the adjacent wavy annular members 2 to each other is used.

As the stent of the blood vessel expansion appliance, an expandable stent, namely, a so-called balloon expandable stent which has a diameter whose dimension is so set that it can be inserted into a lumen inside a living body and which can be expanded when a force spreading radially outward from the inside of the tube is applied thereto is used.

More specifically, as the stent 101, it is possible to use the stents of the above-described embodiments. More specifically, as the stent 101, it is possible to use any of the stents 1, 10, 20, 30, and 40. It is preferable that the area of the wavy element of the stent is 60 to 80% of the area of the peripheral surface of the stent including vacant spaces thereof when the stent is mounted on the balloon 103. The shaft body 102 of the blood vessel expansion appliance 100 of the present invention has a balloon expansion lumen whose one end communicates with the inside of the balloon 103. The stent delivery device 100 has a radiographing member fixed to an outer surface of the shaft body 102 at a position corresponding to the center of the stent or two radiographing members fixed to the outer surface of the shaft body 102 at positions corresponding to one and other ends of the central portion of the stent having a predetermined length.

As shown in Fig. 11, the shaft body 102 of the stent delivery device 100 of this embodiment has a guide wire lumen 115 whose one end is open at a front end of the shaft body 102 and whose other end is open at a rear end of the shaft body 102.

The stent delivery device 100 of the present invention has the tubular shaft body 102, the stent-expanding balloon 103 attached to the front end of the shaft body 102; and the stent 101 mounted on the balloon 3. The shaft body 102 has an inner tube 112, an outer tube 113, and a branch hub 110.

As shown in Fig. 11, the inner tube 112 has the guide wire lumen 115 into which a guide wire is inserted. The length of the inner tube 112 is favorably 100 to 2000mm and more favorably 150 to 1500mm. The outer diameter of the inner tube 112 is favorably 0.1 to 1.0mm and more favorably 0.3 to 0.7mm. The thickness of the inner tube 112 is favorably 10 to 150µm and more favorably 20 to 100µm. The inner tube 112 is inserted into the outer tube 113 to such an extent that the front end of the inner tube 112 projects from the outer tube 113. A balloon-expanding lumen 116 is formed between the outer surface of the inner tube 112 and the inner surface of the outer tube 113 and has a large volume. The front end of the outer tube 113 into which the inner tube 112 is inserted is located a little rearward from the front end of the inner tube 112.

The length of the outer tube 113 is favorably 100 to 2000mm and more favorably 150 - 1500mm. The outer diameter of the outer tube 113 is favorably 0.5 to 1.5mm and more favorably 0.7 to 1.1mm. The thickness of the outer tube 113 is favorably 25 to 200 *µ* m and more favorably 50 to 100 *µ* m.

In the stent delivery device 100 of the embodiment, the outer tube 113 is composed of a front-end side outer tube 113a and a shaft-body side outer tube 113b joined with the front-end side outer tube 113a. The diameter of the front-end side outer tube 113a decreases taperingly in the region forward from the joining position at which the front-end side outer tube 113a and the shaft body side outer tube 113b are joined with each other. The diameter of a portion of the front-end side outer tube 113a forward from the tapered region has a small diameter.

The outer diameter of the front-end side outer tube 113a at its smaller-diameter portion is favorably 0.50 to 1.5mm and more favorably 0.60 to 1.1mm. The outer diameter of the front-end side outer tube 113a at its rear end and that of the shaft-body side outer tube 113b are favorably 0.75 to 1.5mm and more favorably 0.9 to 1.1mm.

The balloon 103 has a front-end side bonding portion 103a and a rear-end side bonding portion 103b. The front-end side bonding portion 103a is fixed to the inner tube 112 at a position a little rearward from the front end thereof. The rear-end side bonding portion 103b is fixed to the front end of the outer tube 113. The balloon 103 communicates with the balloon-expanding lumen 116 at a position in the vicinity of the proximal end thereof.

A material having a certain degree of flexibility can be preferably used for the inner tube 112 and the outer tube 113. It is favorable to use thermoplastic resins such as polyolefin (for example, polyethylene, polypropylene, ethylene-propylene copolymer, ethylene-vinyl acetate copolymer), polyvinyl chloride, polyamide elastomer, and polyurethane; silicone rubber; and latex rubber. It is more favorable to use the thermoplastic resins. Polyolefin is most favorable of the thermoplastic resins.

As shown in Fig. 12, the balloon 103 is foldable. When the balloon 103 is not expanded, it can be folded over the outer surface of the inner tube 112. As shown in Fig. 13, the balloon 103 has a tubular (preferably, cylindrical) expandable portion having an approximately uniform diameter so that it is possible to expand the stent 101 to be mounted on the balloon 103. The expandable portion is not necessarily cylindrical but may be polygonal. As described above, the front-end side bonding portion 103a of the balloon 103 is liquid-tightly bonded to the inner tube 112, and the rear-end side bonding portion 103b thereof is liquid-tightly bonded to the front end of the outer tube 113 with an adhesive agent or by thermal fusion. The balloon 103 tapers between the expandable portion and each of the bonding portions 103a and 103b.

An expansion space 103c is formed between the inner surface of the balloon 103 and the outer surface of the inner tube 112. The entire circumference of the expansion space 103c communicates with the balloon-expanding lumen 116 at the rear end of the expansion space 103c. Because the expansion space 103c communicates with the balloon-expanding lumen 116 having a comparatively large volume, it is easy to inject an expansion fluid into the balloon 103 through the balloon-expanding lumen 116.

Materials having a certain degree of flexibility can be preferably used for the balloon 103. It is favorable to use thermoplastic resins such as polyolefin (for example, polyethylene, polypropylene, ethylene-propylene copolymer, ethylene-vinyl acetate copolymer, crosslinked ethylene-vinyl acetate copolymer), polyvinyl chloride, polyamide elastomer, polyurethane, polyester (for example, polyethylene terephthalate), polyarylane sulfide (for example, polyphenylene sulfide), silicone rubber, and latex rubber. It is particularly favorable to use an extensible material. A biaxially oriented material can be preferably used for the balloon 103 because of its high degree of strength and expansion.

Regarding the size of the balloon 103, the outer diameter of the expanded cylindrical portion (expandable portion) thereof is set favorably to the range of 2 to 4 mm and more favorably 2.5 to 3.5 mm. The length of the balloon 103 is set favorably to the range of 10 to 50 mm and more favorably in the range of 20 to 40 mm. The outer diameter of the front-end side bonding portion 103a is set favorably to the range of 0.9 to 1.5 mm and more favorably in the range of 1 to 1.3 mm. The length of the front-end side bonding portion 103a is set favorably to the range of 1 to 5 mm and more favorably 1 to 1.3 mm. The outer diameter of the rear-end side bonding portion 103b is set favorably to the range of 1 to 1.6 mm and more favorably 1.1 to 1.5 mm. The length of the rear-end side bonding portion 103b is set favorably to the range of 1 to 5 mm and more favorably in the range of 2 to 4 mm.

As shown in Fig. 12, the blood vessel expansion appliance 100 has two radiographing members 117, 118 fixed to the outer surface of the shaft body 102 at positions corresponding to one and other ends of the cylindrical portion (expandable portion) of the stent, when the stent is expanded. Further the blood vessel expansion appliance 100 may have two radiographing members fixed to the outer surface of the shaft body (in this embodiment, inner tube 112) 102 at positions corresponding to one and other ends of the central portion of the stent 101 having a predetermined length. Further the blood vessel expansion appliance 100 may have one radiographing member fixed to the outer surface of the shaft body 102 at a position corresponding to the central portion of the stent 101.

The radiographing members 117 and 118 are preferably in the shape of a ring having a predetermined length or a coiled wire. It is preferable that the radiographing members 117 and 118 are made of gold, platinum, tungsten or alloys thereof or a silver-palladium alloy.

The stent 101 is mounted on the balloon 103, with the stent covering the folded balloon 103. The stent is formed by processing a metal pipe having an inner outer diameter smaller than the inner diameter of the stent at the time when the stent is expanded and larger than the outer diameter of the folded balloon. The balloon is inserted into the formed stent, and a force is uniformly applied to the outer surface of the stent to decrease the diameter of the stent. In this manner, the production of the stent is completed. That is, production of the stent 101 is completed when the stent 101 is mounted on the balloon by compressing the stent.

A linear rigidity-imparting member (not shown) may be inserted between the inner tube 112 and the outer tube 113, namely, into the balloon-expanding lumen 116. The rigidity-imparting member prevents excess bending of the body 102 of the stent delivery device 100 at bent portions of blood vessels without much deteriorating the flexibility of the stent delivery device 100 and facilitates the insertion of the frond end of the stent delivery device 100 into the bent portions of blood vessels. It is preferable that the diameter of the frond end of the rigidity-imparting member is set smaller than those of the other portions thereof by grinding or the like. It is preferable that front end of the small-diameter portion of the rigidity-imparting member extends to the vicinity of the front end of the outer tube of the body of the stent delivery device 100. It is preferable that the rigidity-imparting member consists of a metal wire having a diameter 0.05 to 1.50 mm and more favorably 0.10 to 1.00 mm. The rigidity-imparting member 133 is made of favorably an elastic metal such as stainless steel or a super elastic alloy and more favorably high-strength stainless steel for a spring or a wire of the super elastic alloy.

As shown in Fig. 11; the stent delivery device 100 of this embodiment has a branched hub 110 fixed to the rear end thereof. The branched hub 110 has an inner-tube hub, fixed to the inner tube 112, which has a guide wire introducing opening 109 communicates with the guide wire lumen 115 and forming a guide wire port; and an outer-tube hub, fixed to the outer tube 113, which communicates with the balloon-expanding lumen 116 and has an injection port 111. The outer-tube hub and the inner-tube hub are fixed to each other. As the material of the branched hub 110, thermoplastic resin such as polycarbonate, polyamide, polysulfone, polyacrylate, and methacrylate-butylene-stylene copolymer can be preferably used.

The construction of the stent delivery device is not limited to the above-described one. For example, the stent delivery device may have a guide wire insertion opening, communicating with the guide wire lumen, disposed at a central portion thereof.

### Industrial Applicability

In the stent of the present invention, said wavy annular member has the parallel straight-line portions extended in parallel with the axis of said stent before and after said stent expands. Therefore it is possible to restrain the axial length of the stent from becoming short when the stent expands. Further before and after said stent expands, the connection portion connects ends of said parallel straight-line portions kept in parallel with the axis of said stent. Therefore it is possible to prevent the apexes of the bent portions of the adjacent wavy annular members and thus improve the flexibility (follow-up performance for organs) when the stent contracts and expands and also possible to provide the stent with a sufficient force of expanding organs when the stent expands and in addition uniformly expand the stent.

## Claims

1. A stent (1) formed substantially as a tube which has a form in which a plurality of wavy annular members (2) is arranged adjacently to each other in an axial direction of said stent (1) with said adjacent wavy annular members (2) connected with each other, has a diameter whose dimension is so set that said stent (1) can be inserted into a lumen inside a living body, and can be expanded when a force spreading radially from an inside of said tube is applied to said stent, wherein said wavy annular member (2) has parallel straight-line portions (11) extended in parallel with an axis of said stent (1) when said stent (1) is compressed and at a stent-manufacturing time; and said stent (1) has connection portions (3) each connecting opposed ends of said parallel straight-line portions (11) of said adjacent wavy annular members (2) to each other,
wherein two parallel straight-line portions (11) connected to each other with said connection portion (3) are almost aligned to each other,
**characterized in that**
there are two connection portions (3) connecting adjacent wavy annular members (2) to each other, wherein the connecting portions (3) are provided at opposed positions,
wherein said connection portions (3) adjacent to each other in said axial direction of said stent (1) are shifted from each other in a direction orthogonal to said axis of said stent (1),
wherein said wavy annular member (2) includes a plurality of modified M-shaped linear portions (20), continuous with each other, each of which has four linear portions composed of said parallel straight-line portion (11), a first inclined straight-line portion (12) which is connected with one end of said parallel straight-line portion (11) through a bent portion (15) and becomes oblique to said axis of said stent (1) at a predetermined angle at least when said stent (1) expands, an inclined curved line portion (13) which is connected with one end of said first inclined straight-line portion (12) through a bent portion (16) and extended obliquely at a predetermined angle to said axis of said stent (1), and a second inclined straight-line portion (14) connected with one end of said inclined curved line portion (13) through a bent portion (17) and becomes oblique to said axis of said stent (1) at a predetermined angle at least when said stent (1) expands.

2. A stent (1) according to claim 1, wherein ends of said parallel straight-line portions (11) of said adjacent wavy annular members (2) are proximate to each other and connected to each other with short connection portions (3).

3. A stent (1) according to claim 1 or 2, wherein said connection portions (3) are so disposed that said connection portions (3) are not continuous with each other in said axial direction of said stent (1).

4. A stent (1) according to any one of claims 1 through 3, wherein said stent (1) has a plurality of connection portions (3) connecting said adjacent wavy annular members (2) to each other.

5. A stent (1) according to any one of claims 1 through 4, wherein said wavy annular member (2) has said parallel straight-line portion (11), an inclined straight-line portion (12) which becomes oblique to said axis of said stent (1) at a predetermined angle at least when said stent (1) expands, and an inclined curved line portion (13) which becomes oblique to said axis of said stent (1) at a predetermined angle at least when said stent (1) expands.

6. A stent (1) according to any one of claims 1 through 5, wherein said wavy annular member (2) includes a plurality of modified M-shaped linear portions (20), continuous with each other, each of which has four linear portions composed of said parallel straight-line portion (11); a first inclined straight-line portion (12) which is connected with one end of said parallel straight-line portion (11) through a bent portion (15), is almost parallel with said axis of said stent (1), and becomes oblique to said axis of said stent (1) at a predetermined angle when said stent (1) expands; an inclined curved line portion (13) which is connected with one end of said first inclined straight-line portion (12) through a bent portion and extended obliquely at a predetermined angle to said axis of said stent (1); and a second inclined straight-line portion (14) which is connected with one end of said inclined curved line portion (13) through a bent portion (17), is almost parallel with said axis of said stent (1), and becomes oblique to said axis of said stent (1) at a predetermined angle when said stent (1) expands.

7. A stent according to any one of claims 1 through 6, wherein a length of said parallel straight-line portion is set shorter than that of said first inclined straight-line portion, said inclined curved line portion, and said second inclined straight-line portion.

8. A stent (1) according to any one of claims 1 through 7, wherein said bent portion (15) connecting said one end of said parallel straight-line portion (15) of said wavy annular member (2) and said first inclined straight-line portion (12) thereof to each other is rounded.

9. A stent (1) according to claim 8wherein said bent portion connecting said other end (18) of said parallel straight-line portion (11) of said wavy annular member (2) and said second inclined straight-line portion (14) thereof to each other is rounded.

10. A stent (1) according to any one of claims 1 through 9, wherein at an expansion time of said stent (1), a ratio (B/ A) of an internal angle B formed between said first inclined straight-line portion (12) and said inclined curved line portion to an internal angle A formed between said parallel straight-line portion (11) and said first inclined straight-line portion (12) is set to not less than 1.8.

11. A stent according to any one of claims 1 through 10, wherein at said expansion time of said stent, a ratio (D/ C) of an internal angle D formed between said second inclined straight-line portion (14) and said inclined curved line portion (13) to an internal angle C formed between said parallel straight-line portion (11) and said second inclined straight-line portion(14) is set to not less than 1.8.

12. A stent (1) according to any one of claims 1 through 11, wherein said wavy annular members (2) have a plurality of one-end side bent portions (15, 17) each having an apex at one-end side of said stent (1) in said axial direction thereof and a plurality of other-end side bent portions each having an apex at the other-end side of said stent (1) in said axial direction thereof; and said apex of at least one of said one-end side bent portions (17) of said wavy annular member (2) penetrates to some extent into a space formed between said other-end side bent portions (16) of said adjacent wavy, annular member (2); and said apex of at least one of said other-end side bent portions (16) of said wavy annular member (2) penetrates to some extent into a space formed between said one-end side bent portions (17) of said adjacent wavy annular member (2).

13. A stent delivery device (100) comprising: a tubular shaft body (102); a balloon (103), foldable and expandable, which is disposed at a distal end of said shaft body (102); and a stent (101) which is mounted on said folded balloon (103), with said stent (101) covering said balloon (103) and is expanded owing to expansion of said balloon (103), wherein said stent (101) is a stent according to any one of claims 1 through 12.

## Patentansprüche

1. Stent (1), der im wesentlichen als eine Röhre ausgebildet ist, die eine Form aufweist, in der eine Vielzahl von wellenförmigen ringförmigen Elementen (2) in einer axialen Richtung des Stents (1) benachbart zueinander angeordnet ist, wobei die benachbarten wellenförmigen ringförmigen Elemente (2) miteinander verbunden sind, einen Durchmesser aufweist, dessen Abmessung so festgesetzt ist, dass der Stent (1) in ein Lumen im Inneren eines lebenden Körpers eingeführt werden kann, und ausgedehnt werden kann, wenn eine sich radial von einer Innenseite der Röhre ausbreitende Kraft auf den Stent ausgeübt wird, wobei das wellenförmige ringförmige Element (2) parallele geradlinige Abschnitte (11) aufweist, die sich parallel zu einer Achse des Stents (1) erstrecken, wenn der Stent (1) zusammengedrückt wird und zu einem Zeitpunkt der Herstellung des Stents; und der Stent (1) Verbindungsabschnitte (3) aufweist, die jeweils gegenüberliegende Enden der parallelen geradlinigen Abschnitte (11) der benachbarten wellenförmigen ringförmigen Elemente (2) miteinander verbinden,
wobei zwei parallele geradlinige Abschnitte (11), die durch den Verbindungsabschnitt (3) miteinander verbunden sind, nahezu miteinander ausgerichtet sind,
**dadurch gekennzeichnet, dass**
zwei Verbindungsabschnitte (3) vorhanden sind, die benachbarte wellenförmige ringförmige Elemente (2) miteinander verbinden, wobei die Verbindungsabschnitte (3) an gegenüberliegenden Positionen vorgesehen sind,
wobei die Verbindungsabschnitte (3), die in der axialen Richtung des Stents (1) einander benachbart sind, in einer Richtung, orthogonal zu der Achse des Stents (1), gegeneinander verschoben sind,
wobei das wellenförmige ringförmige Element (2) eine Vielzahl von modifizierten M-förmigen linearen Abschnitten (20) aufweist, die kontinuierlich miteinander sind, von denen jeder vier lineare Abschnitte aufweist, die aus dem parallelen geradlinigen Abschnitt (11), einem ersten geneigten geradlinigen Abschnitt (12), der mit einem Ende des parallelen geradlinigen Abschnitts (11) durch einen gebogenen Abschnitt (15) verbunden ist und zumindest wenn der Stent (1) sich ausdehnt, in einem vorbestimmten Winkel schräg zu der Achse des Stents (1) wird, einem geneigten krummlinigen Abschnitt (13), der mit einem Ende des ersten geneigten geradlinigen Abschnitts (12) durch einen gebogenen Abschnitt (16) verbunden ist und sich schräg in einem vorbestimmten Winkel zu der Achse des Stents (1) erstreckt, und einem zweiten geneigten geradlinigen Abschnitt (14) zusammengesetzt ist, der mit einem Ende des geneigten krummlinigen Abschnitts (13) durch einen gebogenen Abschnitt (17) verbunden ist und zumindest wenn der Stent (1) sich ausdehnt, in einem vorbestimmten Winkel schräg zu der Achse des Stents (1) wird.

2. Stent (1) nach Anspruch 1, wobei die Enden der parallelen geradlinigen Abschnitte (11) der benachbarten wellenförmigen ringförmigen Elemente (2) nahe beieinander liegen und durch kurze Verbindungsabschnitte (3) miteinander verbunden sind.

3. Stent (1) nach Anspruch 1 oder 2, wobei die Verbindungsabschnitte (3) so angeordnet sind, dass die Verbindungsabschnitte (3) in der axialen Richtung des Stents (1) nicht kontinuierlich miteinander sind.

4. Stent (1) nach einem der Ansprüche 1 bis 3, wobei der Stent (1) eine Vielzahl von Verbindungsabschnitten (3) aufweist, welche die benachbarten wellenförmigen ringförmigen Elemente (2) miteinander verbinden.

5. Stent (1) nach einem der Ansprüche 1 bis 4, wobei das wellenförmige ringförmige Element (2) den parallelen geradlinigen Abschnitt (11), einen geneigten geradlinigen Abschnitt (12), der zumindest wenn der Stent (1) sich ausdehnt, in einem vorbestimmten Winkel schräg zu der Achse des Stents (1) wird, und einen geneigten krummlinigen Abschnitt (13) aufweist, der zumindest wenn der Stent (1) sich ausdehnt, in einem vorbestimmten Winkel schräg zu der Achse des Stents (1) wird.

6. Stent (1) nach einem der Ansprüche 1 bis 5, wobei das wellenförmige ringförmige Element (2) eine Vielzahl von modifizierten M-förmigen linearen Abschnitten (20) aufweist, die kontinuierlich miteinander sind, von denen jeder vier lineare Abschnitte aufweist, die aus dem parallelen geradlinigen Abschnitt (11); einem ersten geneigten geradlinigen Abschnitt (12), der mit einem Ende des parallelen geradlinigen Abschnitts (11) durch einen gebogenen Abschnitt (15) verbunden ist, nahezu parallel zu der Achse des Stents (1) ist und wenn der Stent (1) sich ausdehnt, in einem vorbestimmten Winkel schräg zu der Achse des Stents (1) wird; einem geneigten krummlinigen Abschnitt (13), der mit einem Ende des ersten geneigten geradlinigen Abschnitts (12) durch einen gebogenen Abschnitt (16) verbunden ist und sich schräg in einem vorbestimmten Winkel zu der Achse des Stents (1) erstreckt; und einem zweiten geneigten geradlinigen Abschnitt (14) zusammengesetzt ist, der mit einem Ende des geneigten krummlinigen Abschnitts (13) durch einen gebogenen Abschnitt (17) verbunden ist, nahezu parallel zu der Achse des Stents (1) ist und wenn der Stent (1) sich ausdehnt, in einem vorbestimmten Winkel schräg zu der Achse des Stents (1) wird.

7. Stent nach einem der Ansprüche 1 bis 6, wobei eine Länge des parallelen geradlinigen Abschnitts kürzer festgesetzt ist als diejenige des ersten geneigten geradlinigen Abschnitts, des geneigten krummlinigen Abschnitts und des zweiten geneigten geradlinigen Abschnitts.

8. Stent (1) nach einem der Ansprüche 1 bis 7, wobei der gebogene Abschnitt (15), der das eine Ende des parallelen geradlinigen Abschnitts (15) des wellenförmigen ringförmigen Elements (2) und den ersten geneigten geradlinigen Abschnitt (12) davon miteinander verbindet, abgerundet ist.

9. Stent (1) nach Anspruch 8, wobei der gebogene Abschnitt, der das andere Ende (18) des parallelen geradlinigen Abschnitts (11) des wellenförmigen ringförmigen Elements (2) und den zweiten geneigten geradlinigen Abschnitt (14) davon miteinander verbindet, abgerundet ist.

10. Stent (1) nach einem der Ansprüche 1 bis 9, wobei zu einem Zeitpunkt der Ausdehnung des Stents (1) ein Verhältnis (B/A) eines Innenwinkels B, der zwischen dem ersten geneigten geradlinigen Abschnitt (12) und dem geneigten krummlinigen Abschnitt gebildet ist, zu einem Innenwinkel A, der zwischen dem parallelen geradlinigen Abschnitt (11) und dem ersten geneigten geradlinigen Abschnitt (12) gebildet ist, auf nicht weniger als 1,8 festgesetzt ist.

11. Stent nach einem der Ansprüche 1 bis 10, wobei zu einem Zeitpunkt der Ausdehnung des Stents ein Verhältnis (D/C) eines Innenwinkels D, der zwischen dem zweiten geneigten geradlinigen Abschnitt (14) und dem geneigten krummlinigen Abschnitt (13) gebildet ist, zu einem Innenwinkel C, der zwischen dem parallelen geradlinigen Abschnitt (11) und dem zweiten geneigten geradlinigen Abschnitt (14) gebildet ist, auf nicht weniger als 1,8 festgesetzt ist.

12. Stent (1) nach einem der Ansprüche 1 bis 11, wobei die wellenförmigen ringförmigen Elemente (2) eine Vielzahl von gebogenen Abschnitten (15, 17) der einen Endseite, die jeweils einen Scheitelpunkt an einer Endseite des Stents (1) in der axialen Richtung davon aufweisen, und eine Vielzahl von gebogenen Abschnitten der anderen Endseite aufweisen, die jeweils einen Scheitelpunkt an der anderen Endseite des Stents (1) in dessen axialer Richtung aufweisen, und der Scheitelpunkt von mindestens einem der gebogenen Abschnitte (17) der einen Endseite des wellenförmigen ringförmigen Elements (2) bis zu einem gewissen Grad in einen Raum eindringt, der zwischen den gebogenen Abschnitten (16) der anderen Endseite des benachbarten wellenförmigen ringförmigen Elements (2) ausgebildet ist; und der Scheitelpunkt von mindestens einem der gebogenen Abschnitte (16) der anderen Endseite des wellenförmigen ringförmigen Elements (2) bis zu einem gewissen Grad in einen Raum eindringt, der zwischen den gebogenen Abschnitten (17) der einen Endseite des benachbarten wellenförmigen ringförmigen Elements (2) ausgebildet ist.

13. Stenteinführvorrichtung (100), umfassend: einen röhrenförmigen Schaftkörper (102); einen Ballon (103), faltbar und ausdehnbar, der an einem distalen Ende des Schaftkörpers (102) angeordnet ist; und einen Stent (101), der an dem gefalteten Ballon (103) angebracht ist, wobei der Stent (101) den Ballon (103) bedeckt und aufgrund der Ausdehnung des Ballons (103) ausgedehnt wird, wobei der Stent (101) ein Stent nach einem der Ansprüche 1 bis 12 ist.

## Revendications

1. Stent (1) formé substantiellement comme un tube qui présente une forme dans laquelle une pluralité d'éléments annulaires ondulés (2) sont disposés de façon adjacente les uns aux autres dans une direction axiale dudit stent (1), lesdits éléments annulaires ondulés (2) adjacents étant reliés les uns aux autres, présente un diamètre dont la dimension est définie de telle sorte que ledit stent (1) peut être introduit dans une lumière à l'intérieur d'un corps vivant, et peut être déployé quand une force se diffusant radialement depuis un intérieur dudit tube est appliquée audit stent, dans lequel ledit élément annulaire ondulé (2) présente des parties en ligne droite parallèles (11) s'étendant parallèlement à un axe dudit stent (1) lorsque ledit stent (1) est comprimé et à un moment de fabrication du stent ; et ledit stent (1) présente des parties de liaison (3) reliant chacune, les unes aux autres, les extrémités opposées desdites parties en ligne droite parallèles (11) desdits éléments annulaires ondulés (2) adjacents,
dans lequel deux parties en ligne droite parallèles (11) reliées mutuellement à ladite partie de liaison (3) sont quasiment alignées l'une avec l'autre,
**caractérisé en ce que**
sont prévues deux parties de liaison (3) reliant les uns aux autres des éléments annulaires ondulés (2) adjacents, dans lequel les parties de liaison (3) sont prévues en des positions opposées,
dans lequel lesdites parties de liaison (3) adjacentes les unes aux autres dans ladite direction axiale dudit stent (1) sont décalées les unes des aux autres dans une direction orthogonale audit axe dudit stent (1),
dans lequel ledit élément annulaire ondulé (2) comporte une pluralité de parties linéaires modifiées en forme de M (20), continues les unes avec les autres, chacune d'elles présentant quatre parties linéaires composées de ladite partie en ligne droite parallèle (11), une première partie en ligne droite inclinée (12) qui est reliée à une extrémité de ladite partie en ligne droite parallèle (11) par le biais d'une partie courbée (15) et devient oblique par rapport audit axe dudit stent (1) à un angle prédéterminé au moins lors de l'expansion dudit stent (1), une partie en ligne courbe inclinée (13) qui est reliée à une extrémité de ladite première partie en ligne droite inclinée (12) par le biais d'une partie courbée (16) et s'étend de façon oblique à un angle prédéterminé par rapport audit axe dudit stent (1), et une seconde partie en ligne droite inclinée (14) reliée à une extrémité de ladite partie en ligne courbe inclinée (13) par le biais d'une partie courbée (17) et devient oblique par rapport audit axe dudit stent (1) à un angle prédéterminé au moins lors de l'expansion dudit stent (1).

2. Stent (1) selon la revendication 1, dans lequel les extrémités desdites parties en ligne droite parallèles (11) desdits éléments annulaires ondulés (2) adjacents sont proches les unes des autres et sont reliées les unes aux autres par de courtes parties de liaison (3).

3. Stent (1) selon la revendication 1 ou 2, dans lequel lesdites parties de liaison (3) sont disposées de telle sorte que lesdites parties de liaison (3) ne sont pas continues les unes par rapport aux autres dans ladite direction axiale dudit stent (1).

4. Stent (1) selon l'une quelconque des revendications 1 à 3, dans lequel ledit stent (1) présente une pluralité de parties de liaison (3) reliant les uns aux autres lesdits éléments annulaires ondulés (2) adjacents.

5. Stent (1) selon l'une quelconque des revendications 1 à 4, dans lequel ledit élément annulaire ondulé (2) présente ladite partie en ligne droite parallèle (11), une partie en ligne droite inclinée (12) qui devient oblique par rapport audit axe dudit stent (1) à un angle prédéterminé au moins lors de l'expansion dudit stent (1), et une partie en ligne courbe inclinée (13) qui devient oblique par rapport audit axe dudit stent (1) à un angle prédéterminé au moins lors de l'expansion dudit stent (1).

6. Stent (1) selon l'une quelconque des revendications 1 à 5, dans lequel ledit élément annulaire ondulé (2) comporte une pluralité de parties linéaires modifiées en forme de M (20), continues les unes avec les autres, chacune d'elles présentant quatre parties linéaires composées de ladite partie en ligne droite parallèle (11) ; une première partie en ligne droite inclinée (12) qui est reliée à une extrémité de ladite partie en ligne droite parallèle (11) par le biais d'une partie courbée (15), est quasiment parallèle audit axe dudit stent (1), et devient oblique par rapport audit axe dudit stent (1) à un angle prédéterminé lors de l'expansion dudit stent (1) ; une partie en ligne courbe inclinée (13) qui est reliée à une extrémité de ladite première partie en ligne droite inclinée (12) par le biais d'une partie courbée et s'étend de façon oblique à un angle prédéterminé par rapport audit axe dudit stent (1) ; et une seconde partie en ligne droite inclinée (14) qui est reliée à une extrémité de ladite partie en ligne courbe inclinée (13) par le biais d'une partie courbée (17), est quasiment parallèle audit axe dudit stent (1), et devient oblique par rapport audit axe dudit stent (1) à un angle prédéterminé lors de l'expansion dudit stent (1).

7. Stent selon l'une quelconque des revendications 1 à 6, dans lequel une longueur de ladite partie en ligne droite parallèle est définie plus courte que celle de ladite première partie en ligne droite inclinée, de ladite partie en ligne courbe inclinée, et de ladite seconde partie en ligne droite inclinée.

8. Stent (1) selon l'une quelconque des revendications 1 à 7, dans lequel ladite partie courbée (15) reliant entre elles ladite première extrémité de ladite partie en ligne droite parallèle (15) dudit élément annulaire ondulé (2) et ladite première partie en ligne droite inclinée (12) de celui-ci est arrondie.

9. Stent (1) selon la revendication 8 dans lequel ladite partie courbée reliant entre elles ladite autre extrémité (18) de ladite partie en ligne droite parallèle (11) dudit élément annulaire ondulé (2) et ladite seconde partie en ligne droite inclinée (14) de celui-ci est arrondie.

10. Stent (1) selon l'une quelconque des revendications 1 à 9, dans lequel, lors de l'expansion dudit stent (1), un rapport (B/A) d'un angle interne B formé entre ladite première partie en ligne droite inclinée (12) et ladite partie en ligne courbe inclinée à un angle interne A formé entre ladite partie en ligne droite parallèle (11) et ladite première partie en ligne droite inclinée (12) est défini pour ne pas être inférieur à 1,8.

11. Stent (1) selon l'une quelconque des revendications 1 à 10, dans lequel, lors de ladite expansion dudit stent, un rapport (D/C) d'un angle interne D formé entre ladite seconde partie en ligne droite inclinée (14) et ladite partie en ligne courbe inclinée (13) à un angle interne C formé entre ladite partie en ligne droite parallèle (11) et ladite seconde partie en ligne droite inclinée (14) est défini pour ne pas être inférieur à 1,8.

12. Stent (1) selon l'une quelconque des revendications 1 à 11, dans lequel lesdits éléments annulaires ondulés (2) présentent une pluralité de parties courbées côté première extrémité (15, 17) présentant chacune un sommet au niveau d'un côté première extrémité dudit stent (1) dans ladite direction axiale de celui-ci et une pluralité de parties courbées côté autre extrémité présentant chacune un sommet au niveau du côté autre extrémité dudit stent (1) dans ladite direction axiale de celui-ci ; et ledit sommet d'au moins l'une desdites parties courbées côté première extrémité (17) dudit élément annulaire ondulé (2) pénètre dans une certaine mesure dans un espace formé entre lesdites parties courbées côté autre extrémité (16) dudit élément annulaire ondulé adjacent (2) ; et ledit sommet d'au moins l'une desdites parties courbées côté autre extrémité (16) dudit élément annulaire ondulé (2) pénètre dans une certaine mesure dans un espace formé entre lesdites parties courbées côté première extrémité (17) dudit élément annulaire ondulé (2) adjacent.

13. Dispositif d'introduction de stent (100) comprenant : un corps de tige tubulaire (102) ; un ballonnet (103), compressible et expansible, qui est disposé au niveau d'une extrémité distale dudit corps de tige (102) ; et un stent (101) qui est monté sur ledit ballonnet comprimé (103), ledit stent (101) couvrant ledit ballonnet (103) et étant déployé sous l'effet de l'expansion dudit ballonnet (103), dans lequel ledit stent (101) est un stent selon l'une quelconque des revendications 1 à 12.
